(19) Europäisches Patentamt
European Patent Office
Office européen des brevets

(11) EP 1 848 530 B1

(12) FASCICULE DE BREVET EUROPEEN

(45) Date de publication et mention de la délivrance du brevet:
**03.11.2010 Bulletin 2010/44**

(21) Numéro de dépôt: **06709118.1**

(22) Date de dépôt: **18.01.2006**

(51) Int Cl.:
***B01J 23/00*** *(2006.01)*
***B01J 23/28*** *(2006.01)*
***B01J 23/20*** *(2006.01)*
***B01J 23/18*** *(2006.01)*
***B01J 27/057*** *(2006.01)*
***C07C 45/35*** *(2006.01)*
***C07C 51/25*** *(2006.01)*

(86) Numéro de dépôt international:
**PCT/FR2006/000111**

(87) Numéro de publication internationale:
**WO 2006/077316 (27.07.2006 Gazette 2006/30)**

# (54) PROCEDE DE PREPARATION DE L'ACIDE ACRYLIQUE COMPRENANT UNE OXYDATION PARTIELLE DU PROPANE EN PROPYLENE

VERFAHREN ZUR HERSTELLUNG VON ACRYLSÄURE MIT PARTIALOXIDATION VON PROPAN ZU PROPYLEN

METHOD FOR PREPARING ACRYLIC ACID INVOLVING A PARTIAL OXIDATION OF PROPANE INTO PROPYLENE

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **21.01.2005 FR 0500643**

(43) Date de publication de la demande:
**31.10.2007 Bulletin 2007/44**

(73) Titulaire: **ARKEMA FRANCE**
**92700 Colombes (FR)**

(72) Inventeurs:
- **DUBOIS, Jean-Luc**
**F-69390 Millery (FR)**
- **SERREAU, Stéphanie**
**F-69600 Oullins (FR)**
- **DESDEVISES, Fabienne**
**F-69390 Millery (FR)**

(74) Mandataire: **Hirsch & Associés**
**58, avenue Marceau**
**75008 Paris (FR)**

(56) Documents cités:
**EP-A- 0 253 409**
**EP-A- 0 274 681**
**EP-A- 0 293 224**
**BE-A1- 896 443**
**US-A1- 2003 187 298**
**US-B1- 6 492 548**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention concerne la préparation de l'acide acrylique à partir du propylène comprenant un recyclage des gaz avec une étape d'oxydation de propane en propylène.

La production d'acide acrylique consiste généralement dans une oxydation en 2 étapes, d'une part une première étape d'oxydation du propylène en acroléine et d'autre part une deuxième étape d'oxydation de l'acroléine en acide acrylique.

Cependant la productivité est limitée par des contraintes importantes telles que l'inflammabilité et les dangers d'explosion du mélange propylène / air / azote / vapeur d'eau, l'évacuation du réacteur de la quantité de chaleur produite, ainsi que la sensibilité du catalyseur aux fortes teneurs en propylène. Il est ainsi avantageux d'introduire du propane dans le flux gazeux comprenant le propylène, ce qui permet d'éliminer une partie de la chaleur de réaction et en conséquence d'augmenter la teneur en propylène.

La demande de brevet européen EP 293 224 décrit l'oxydation en 2 étapes du propylène en acide acrylique et notamment la réaction d'oxydation du propylène en acroléine en présence de 5 à 70% en volumes d'un hydrocarbure aliphatique saturé (1 à 5C) comme par exemple le propane, et 3 à 50% en volumes de dioxyde de carbone utilisés comme gaz inertes. Les hydrocarbures aliphatiques saturés mis en oeuvre présentent une chaleur spécifique au voisinage de 300°C et à pression constante, plus élevée que celle de l'azote ou de l'air. Ainsi, le gaz ajouté est capable d'absorber, en partie, la chaleur produite par la réaction d'oxydation. De ce fait il est possible d'augmenter la teneur en propylène dans le gaz de réaction et de produire une quantité plus importante d'acide acrylique. Commercialement parlant, il peut être envisagé de préparer les gaz de départ en utilisant les gaz récupérés après la 1ère étape de réaction. Cependant rien ne précise si une conversion du propane introduit est réellement mise en oeuvre. De plus il n'était pas aisé de convertir, à l'échelle industrielle, le propane en propylène en évitant la formation de nombreux sous produits de réaction pouvant nuire aux opérations suivantes.

Le brevet US 6,492,548 décrit la conversion du propane en propylène, puis en acroléine et en acide acrylique. La présence de propane dans la phase d'oxydation du propylène en acroléine, améliore l'efficacité de cette phase de réaction. A l'issue de la réaction de préparation de l'acroléine, il est avantageux de recycler le propane dans un réacteur destiné à son oxydation en propylène, produisant, de préférence, de faibles taux de conversion du propane et de fortes sélectivités en propylène. L'oxydation du propane en propylène est conduite en présence d'un catalyseur comme par exemple un oxyde métallique mixte comprenant comme éléments essentiels du molybdène, du vanadium, du tellure, et au moins un autre élément choisi parmi le niobium, le tungxtène, le titane ... ou l'antimoine. La réaction d'oxydation du propane est en général conduite à une température comprise entre 200 et 550°C. La réaction de conversion du propylène en acroléine est conduite en milieu catalytique à une température élevée. De nombreux sous produits sont formés au cours de la réaction, qui doivent être séparés. Selon l'enseignement de la figure 2, à la sortie de l'unité de récupération de l'acide acrylique, le courant de gaz n'ayant pas réagi, constitué de propane, de propylène, d'oxygène et de monoxide et dioxide de carbone (et éventuellement d'azote) est envoyé dans un courant de recyclage, puis comprimé et réintroduit, en continu, dans le procédé de conversion propane → propylène → acroléine → acide acrylique. Cependant il est connu que les catalyseurs utilisés pour la conversion du propane en propylène (oxydes de MoVNb) conduisent à la formation d'acide acrylique en plus du propylène. L'acide acrylique formé à cette étape est dirigé vers le réacteur de conversion du propylène en acroléine, où il peut avoir un effet négatif sur la réaction.

Il est connu que l'oxydation catalytique du propane peut conduire à de très nombreux produits de réaction, en fonction des conditions opératoires utilisées. L. Luo, J. A. Labinger et M. E. Davis, J. of Catalysis, 200, 222-231 (2001) ont décrit les différentes voies d'oxydation catalytique partielle du propane en présence d'oxydes métalliques, qui peuvent être résumées par le schéma ci-après, comprenant 3 grandes voies réactionnelles :

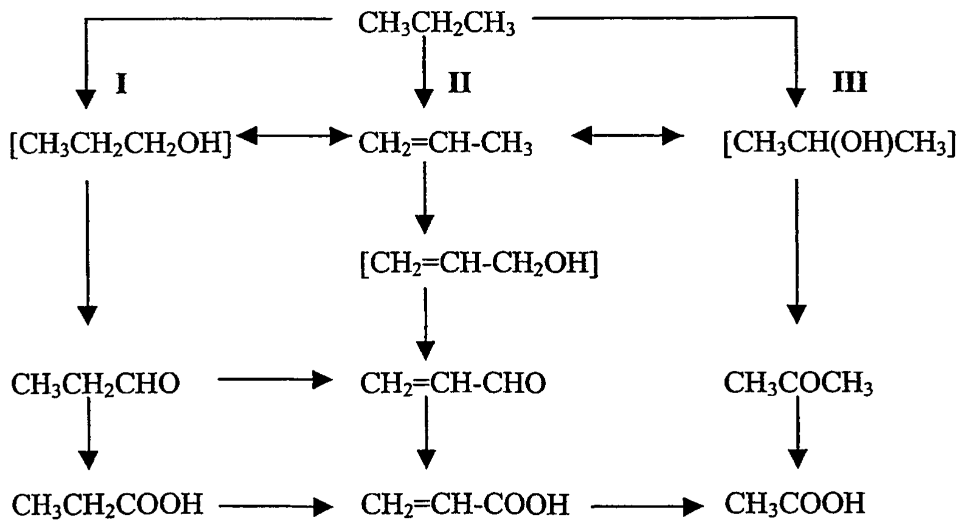


L'orientation spécifique vers l'un ou l'autre des produits d'oxydation, avec des rendements industriellement performants, requiert la fixation très précise des paramètres de mise en oeuvre de ladite oxydation. Il est aisé de comprendre que de nombreux sous produits de réaction peuvent se former, ces derniers pouvant s'avérer être un handicap dans le déroulement de la réaction ou pour l'isolement du produit souhaité.

Il a été mis en évidence, que la présence d'acide acrylique pouvait géner considérablement l'étape de conversion du propylène en acroléine. De ce fait, dans la préparation industrielle de l'acide acrylique, les opérations de recyclage des gaz n'ayant pas réagi, peuvent s'avérer ne plus être véritablement un avantage mais un inconvénient important du fait de quantités d'acide acrylique mal séparées du courant gazeux et qui se trouvent présentes lors de la conversion du propylène en acroléine.

Il a été maintenant trouvé, et c'est ce qui fait l'objet de la présente invention, que la préparation de l'acide acrylique par oxydation du propylène puis de l'acroléine pouvait être mise en oeuvre avec recyclage des gaz n'ayant pas réagi et plus particulièrement avec oxydation partielle du propane, en effectuant cette oxydation, en parallèle, à l'issue de l'étape de récupération de l'acide acrylique, puis en retournant vers le réacteur de conversion du propylène, un gaz riche en propane et en propylène ayant subi un second passage dans la colonne de récupération de l'acide acrylique, ainsi que cela est montré sur la figure 1.

Cette amélioration du procédé de préparation de l'acide acrylique à partir du propylène consiste dans une conversion partielle, en propylène, du propane utilisé comme gaz inerte dans la première étape d'oxydation du propylène en acroléine et récupéré à l'issue de la deuxième étape de conversion en acide acrylique, de manière à permettre d'augmenter la teneur en propylène de départ tout en maintenant des pressions partielles en propane suffisamment élevées. Un avantage important de ce procédé est de débarrasser le flux gazeux, de l'acide acrylique et d'éviter l'introduction de cet acide dans le réacteur d'oxydation du propylène en acroléine. Un autre avantage est d'accroître le rendement en acide acrylique (sous produit majoritaire de la réaction d'oxydation partielle du propane) et également de limiter la formation de sous-produits secondaires comme l'acide propionique ou l'acétone.

Selon l'invention, le propane est soumis à une conversion partielle en propylène, dans un réacteur placé en parallèle de la colonne d'adsorption destinée à la récupération de l'acide acrylique. Cette conversion partielle a lieu en présence d'un catalyseur constitué de mélange d'oxydes et dans des conditions opératoires spécifiques permettant de limiter la formation des sous-produits comme notamment l'acide propionique et l'acétone et d'obtenir le propylène comme produit majoritaire. Le flux gazeux est alors réintroduit dans la colonne d'absorption utilisée pour la récupération de l'acide acrylique, puis dirigé vers le réacteur de conversion du propylène en acroléine.

Selon l'invention, le procédé d'oxydation partielle du propane en propylène est mis en oeuvre à température élevée (300 à 500°C et de préférence supérieure à 380°C, jusqu'à 450°C) et à des vitesses volumiques horaires élevées VVH (débit de gaz de réaction / volume de catalyseur). On opère avantageusement à des VVH supérieures à 10000 $h^{-1}$ et de préférence comprises entre des valeurs supérieures à 10 000 $h^{-1}$, et 20 000 $h^{-1}$.

Selon l'invention, le catalyseur utilisé pour l'oxydation partielle du propane en propylène, est un mélange d'oxydes contenant des éléments choisis parmi le molybdène, le vanadium, le tellure ou l'antimoine, le niobium ou le tantale, le silicium répondant à la structure :

$$Mo_1V_a(\text{Te ou Sb})_b(\text{Nb ou Ta})_cSi_dO_x \qquad (I)$$

dans laquelle :

- a est compris entre 0,006 et 1, bornes incluses ;
- b est compris entre 0,006 et 1, bornes incluses ;
- c est compris entre 0,006 et 1, bornes incluses ;
- d est compris entre 0 et 3,5, bornes incluses ; et
- x est la quantité d'oxygène lié aux autres éléments et dépend de leurs états d'oxydation.

Le catalyseur de formule (Ia)

$$Mo_1V_aSb_bNb_cSi_dO_x \qquad (Ia)$$

dans laquelle a, b, c, d et x sont définis comme ci-dessus, est tout particulièrement préféré.

Plus particulièrement préférés sont aussi les catalyseurs (I) ou (Ia) pour lesquels :

- a est compris entre 0,01 et 0,6, bornes incluses ;
- b est compris entre 0,01 et 0,5, bornes incluses ;
- c est compris entre 0,006 et 0,3, bornes incluses ;
- d est compris entre 0 et 2, bornes incluses ; et

x est la quantité d'oxygène lié aux autres éléments et dépend de leurs états d'oxydation.

Le catalyseur peut être mis en forme sur un support inerte, selon les techniques connues de l'homme de l'art et appliquées pour les réacteurs à lit fixe. Par exemple, il peut être mis en forme par extrusion, pastillage, enrobage, imprégnation et de préférence par enrobage.

Les oxydes des différents métaux entrant dans la composition du catalyseur de formule (I) peuvent être utilisés comme matières premières dans la préparation de ce catalyseur, mais les matières premières ne sont pas limitées aux oxydes ; parmi les matières premières pouvant être utilisées, on peut citer, à titre non limitatif :

- dans le cas du molybdène, le molybdate d'ammonium, le paramolybdate d'ammonium, l'hepta-molybdate d'ammonium, l'acide molybdique, les halogènures ou oxyhalogénures de molybdène tels que $MoCl_5$, les composés organométalliques du molybdène comme les alkoxydes de molybdène tels que $Mo(OC_2H_5)_5$, le molybdényle d'acétylacétone ;
- dans le cas du vanadium, le métavanadate d'ammonium, les halogénures ou oxyhalogénures de vanadium tels que $VCl_4$, $VCl_5$ ou $VOCl_3$, les composés organométalliques du vanadium comme les alkoxydes de vanadium tels que $VO(OC_2H_5)_3$;
- dans le cas de l'antimoine par exemple de l'oxyde d'antimoine (trioxyde d'antimoine), notamment la variété Senarmontite, le sulfate d'antimoine ($Sb_2(SO_4)_3$) ou un chlorure d'antimoine (trichlorure d'antimoine, pentachlorure d'antimoine) ;
- dans le cas du tellure, le tellure, l'acide tellurique, $TeO_2$ ;
- dans le cas du niobium, l'acide niobique, le tartrate de niobium, l'hydrogéno-oxalate de niobium, le niobiate d'oxo-trioxalato-ammonium $\{(NH_4)_3[NbO(C_2O_4)_3]\cdot 1{,}5H_2O\}$, l'oxalate de niobium et d'ammonium, l'oxalate de niobium et le tartrate, les halogénures ou oxyhalogénures de niobium tels que $NbCl_3$, $NbCl_5$ et les composés organométalliques du niobium comme les alkoxydes de niobium tels que $Nb(OC_2H_5)_5$, $Nb(O\text{-}n\text{-}Bu)_5$ ;
- dans le cas du tantale, l'oxalate de tantale ;

et, d'une manière générale, tous les composés susceptibles de former un oxyde par calcination, à savoir, les sels métalliques d'acides organique, les sels métalliques d'acides minéraux, les composés métalliques complexes, etc.

La source de silicium est généralement constituée de silice colloïdale et/ou d'acide polysilicique.

A titre d'exemple, le support du catalyseur est avantageusement la silice, l'alumine, la silice-alumine, la stéatite, une céramique ou le carbure de silicium.

Un mode de préparation des catalyseurs consiste à mélanger sous agitation des solutions aqueuses d'acide niobique ou d'oxalate de tantale, d'acide oxalique, d'heptamolybdate d'ammonium, de métavanadate d'ammonium, d'acide tellurique ou d'oxyde d'antimoine, en ajoutant le cas échéant de la silice colloïdale, puis de préférence précalciner sous air à environ 300-320°C, et calciner sous azote à environ 600°C.

Selon un mode préféré, un procédé de préparation des catalyseurs consiste dans la préparation d'une solution d'acide niobique et d'acide oxalique, ou dans l'utilisation d'une solution d'oxalate de tantale commerciale, puis la préparation d'une solution de molybdène, de vanadium, de tellure ou d'antimoine, suivie du mélange des 2 solutions donnant lieu à la formation d'un gel, séchage du gel obtenu, et précalcination puis calcination.

Selon un procédé particulièrement préféré, le catalyseur peut être préparé en mettant en oeuvre les étapes suivantes :

1) dissolution dans de l'eau d'une source de vanadium, par exemple, du métavanadate d'ammonium, sous agitation et en chauffant éventuellement ;
2) le cas échéant addition à la solution obtenue précédemment d'une source de tellure ou d'antimoine, par exemple l'acide tellurique, ou l'oxyde d'antimoine (en particulier la variété Sénarmontite) ;
3) addition d'une source de molybdène, par exemple, l'heptamolybdate d' ammonium ;
4) réaction de la solution obtenue, sous reflux ;
5) le cas échéant, addition d'un oxydant tel que l'eau oxygénée dans le cas des catalyseurs à l'antimoine ;
6) le cas échéant addition d'une solution préparée en mélangeant, sous chauffage, une source de niobium, par exemple, de l'acide niobique, avec de l'acide oxalique ;
7) réaction du mélange réactionnel sous reflux et de préférence sous atmosphère inerte, jusqu'à l'obtention d'un gel ;
8) séchage du gel obtenu
9) de préférence précalcination du gel ; et
10) calcination du gel, éventuellement précalciné, pour obtenir le catalyseur.

La source de silicium (silice colloïdale et/ou acide polysilicique) est avantageusement ajoutée après l'étape 5). Il est également possible de l'ajouter après les étapes de séchage ou de précalcination.
Dans les alternatives de procédés ci-dessus :

le séchage [par exemple de l'étape 8)] peut être effectué à l'étuve en couche mince, par atomisation, par lyophilisation, par zéodratation, par micro-ondes, etc ;
la précalcination peut être effectuée sous flux d'air à 280-300°C ou sous air statique à 320°C, en lit fluidisé, en four tournant en lit fixe dit aéré, de façon à ce que les grains de catalyseur soient séparés les uns des autres pour éviter qu'ils ne fusionnent lors de la précalcination ou éventuellement lors de la calcination ;
la calcination est de préférence effectuée sous azote très pur et à une température voisine de 600°C, par exemple en four tournant ou en lit fluidisé et pendant une durée qui peut être de 2 heures.

Selon des modes de réalisation préférés de la précalcination, on opère :

- soit à une température inférieure à 300°C sous un débit d'air d'au moins 10 ml/min/g de catalyseur ; notamment à environ 290°C, sous un débit d'air d'environ 50 ml/min/g.
- soit à une température allant de 300 à 350°C sous un débit d'air inférieur à 10 ml/min/g de catalyseur ; notamment à environ 320°C sous un débit d'air inférieur à 10 ml/min/g.

Selon un autre mode de préparation des catalyseurs, on effectue une réaction solide-solide par mélange des sources de métaux puis co-broyage jusqu'à obtention d'un mélange homogène. Le solide est obtenu après chauffage sous pression réduite à une température voisine de 600°C.
Avantageusement on utilise les oxydes des métaux ou le métal lui-même comme source de métaux. Plus préférentiellement le chauffage est mis en oeuvre pendant un temps prolongé (de préférence 3 jours à 1 semaine).
Les catalyseurs préparés selon les procédés décrits ci-avant peuvent se présenter chacun sous la forme de grains généralement de 20 à 300 $\mu$m de diamètre, les grains de chacun des catalyseurs associés étant généralement mélangés avant la mise en oeuvre du procédé selon l'invention. La mise en forme peut être effectuée par atomisation d'un gel ou d'une suspension. Pour une utilisation en lit fixe, les catalyseurs peuvent se présenter sous forme de billes ou de cylindres ou encore de cylindres creux de diamètre 3 à 10 mm, de préférence 5 à 8 mm, enrobés de matière active.

Dispositif d'installation

La figure 1 décrit un dispositif d'installation permettant de mettre en oeuvre le procédé de préparation de l'acide acrylique, avec recyclage des gaz n'ayant pas réagi comprenant une oxydation partielle en parallèle; du propane. Il est entendu que ce dispositif d'installation entre également dans le cadre de la présente invention.
Dans la figure 1, les éléments numérotés de 1 à 7 ont les significations suivantes :

1 : Charge fraîche en propylène/propane
2 : Mélange de réaction (propane, propylène, vapeur d'eau, oxygène)
3 : Réacteur de conversion du propylène en acroléine
4 : Réacteur de conversion de l'acroléine en acide acrylique

5 : colonne d'absorption
6 : port d'ajout d'oxygène
7 : Réacteur de conversion du propane.

L'invention concerne également un dispositif destiné à la préparation de l'acide acrylique comprenant :

a) un premier réacteur d'oxydation du propylène en acroléine [3], alimentant en continu
b) un second réacteur destiné à l'oxydation de l'acroléine en acide acrylique [4], relié à
c) une colonne d'absorption [5] destinée à la récupération de l'acide acrylique, les gaz n'ayant pas réagi étant dirigés vers
d) un réacteur [7] d'oxydation partielle du propane en propylène, placé en parallèle à la sortie de la colonne d'absorption [5], lesdits gaz subissant à la sortie un nouveau passage dans la colonne d'absorption ou dans une colonne similaire, puis
e) un recyclage dans le premier réacteur d'oxydation du propylène [3].

Dans la figure 1, le réacteur [7], placé en parallèle de la colonne d'absorption [5] permettant la récupération de l'acide acrylique, est destiné à l'oxydation partielle du propane en propylène. Le flux gazeux sortant de la colonne [5] est dirigé vers le réacteur [7] contenant un lit de catalyseur à base de mélange d'oxydes. Ce flux gazeux contient principalement du propane, du propylène n'ayant pas réagi, de la vapeur d'eau, du monoxyde de carbone, du gaz carbonique, de l'oxygène résiduel et éventuellement des gaz inertes (argon), de l'acroléine et de l'acétone. Un flux gazeux comprenant un mélange vapeur d'eau / oxygène moléculaire / éventuellement gaz inerte, est également introduit dans le réacteur [7].

De préférence le lit de catalyseur est un lit fixe : notamment lit fixe co-alimenté. Selon une autre alternative, il peut être également envisagé d'utiliser un réacteur à lit fluidisé ou à lit transporté.

Le rapport molaire propane / oxygène moléculaire est supérieur à 1 et de préférence supérieur ou égal à 4. Dans le mélange gazeux mis en oeuvre dans la réaction d'oxydation partielle du propane, la teneur en propane doit être au moins supérieure à 20%, et inférieure à 90%.

Le rapport en volume propane / vapeur d'eau dans le mélange gazeux globalement introduit dans le réacteur [7] n'est pas critique et peut varier dans de larges limites. Il n'est pas indispensable d'introduire de la vapeur d'eau en fortes quantités, celle ci pouvant même être absente du mélange gazeux.

De même, la proportion de gaz inerte, qui peut être de l'hélium, du krypton, un mélange de ces deux gaz, l'argon, ou bien de l'azote, du dioxyde de carbone, etc., n'est pas non plus critique et peut aussi varier dans de larges limites.

Les proportions des constituants du mélange gazeux initial sont généralement les suivantes (en rapports molaires) :

propane / oxygène / gaz inerte (Ar, $N_2$, $CO_2$) / $H_2O$ (vapeur) = 1 /0,05-2/0-12/0,1-10.

De préférence, elles sont de 1 / 0,1-1 / 0-11 / 0,3-6.

La pression dans le réacteur est généralement fixée de $1{,}01.10^4$ à $1{,}01.10^6$ Pa (0,1 à 10 atmosphères), de préférence de $5{,}05.10^4$ à $5{,}05.10^5$ Pa (0,5-5 atmosphères). De préférence lorsque l'on opère en lit fixe, la pression est fixée à $2.10^5$ Pa.

A la sortie du réacteur [7], le flux gazeux riche en propane et en propylène est réintroduit dans la colonne d'absorption [5] (ou éventuellement dans une seconde colonne d'absorption similaire. Dans ce cas les effluents de la seconde colonne peuvent alimenter la première colonne). Dans la/les colonnes d'adsorption l'acide acrylique, sous produit de la réaction d'oxydation dans le réacteur [7], est récupéré. Ainsi le flux gazeux envoyé par recyclage vers le réacteur [3] de conversion du propylène en acroléine, est débarassé de l'acide acrylique résiduel.

Le réacteur [3] de conversion du propylène en acroléine reçoit un mélange d'une charge fraîche propylène / propane [1] et du mélange de réaction recyclé (comprenant propylène / propane / vapeur d'eau / oxygène / éventuellement gaz inertes) [2], ainsi qu'un ajout d'oxygène moléculaire [6]. Principalement, les gaz inertes peuvent être l'azote, le dioxyde de carbone, l'argon ainsi que d'autres gaz amenés par le gaz recyclé, comme le méthane, l'éthane ...

Il est important de sélectionner un catalyseur favorable à la conversion du propylène en acroléine, mais qui soit insensible à la présence de propane. Généralement la réaction est catalysée par un catalyseur comme un molybdate de bismuth, à une température voisine de 320°C et sous une pression de 2.105 Pa. Le catalyseur peut être choisi par exemple parmi les molybdates décrits dans le tableau 2 de la publication de M. Tanimoto, Shokubai, 45(5), 360 (2003).

La charge fraîche propylène / propane [1] peut être une coupe propylène issue d'un vapocraqueur. Dans ce cas la teneur en propane est de l'ordre de 5%. Il est également possible d'utiliser une coupe propylène / propane issue des raffineries de pétrole.

Avantageusement la teneur en propane dans la charge fraîche de propylène / propane doit être au moins 5 %.
La proportion globale de mélange propylène / propane / vapeur d'eau / oxygène / éventuellement gaz inertes, reçue par le réacteur doit être telle que de fortes pressions partielles en propane sont assurées. Cette proportion se situe de préférence dans les limites 5 à 15 / 30 à 50 / 0 à 15 / 5 à 20 / 0 à 50.
Le flux gazeux sortant du réacteur [3] est envoyé dans le réacteur [4] de conversion de l'acroléine en acide acrylique.
Le réacteur [4] reçoit le mélange gazeux issu du réacteur [3] qui est alors oxydé en acide acrylique en présence d'un catalyseur favorable à la conversion de l'acroléine en acide acrylique, mais insensible à la présence de propane. Généralement la réaction est catalysée par un catalyseur tel qu'un oxyde mixte à base de molybdène et de vanadium sous une pression de $2.10^5$ Pa et à une température voisine de 250°C. Le catalyseur peut être choisi par exemple parmi les oxydes mixtes décrits dans le tableau 3 de la publication de M. Tanimoto, Shokubai, 45(5), 360 (2003).
La présente invention présente le grand avantage d'associer une très bonne sélectivité en acide acrylique et une bonne conversion du propylène, du fait d'un recyclage des gaz assurant à la fois de fortes pressions partielles en propane dans le réacteur d'oxydation du propylène en acroléine, une plus forte proportion de propylène introduit et de très faibles proportions d'acide acrylique au niveau de cette première étape d'oxydation. De plus le passage du flux gazeux, issu du réacteur de conversion partielle du propane, dans la colonne d'absorption permettant l'isolement de l'acide acrylique assure un complément du rendement en acide acrylique, ce dernier étant le principal sous produit de réaction dans la conversion partielle du propane en propylène.
Il est entendu que la présente invention concerne également l'utilisation du procédé décrit ci-avant, pour la préparation de l'acide acrylique.
Cet avantage peut être observé en particulier dans les essais qui suivent.

## EXEMPLES

Les exemples suivants illustrent la présente invention sans toutefois en limiter la portée.

### Préparation des catalyseurs d'oxydation partielle du propane en propylène

### Exemple A : Préparation du catalyseur A : $Mo_1V_{0,33}Nb_{0,11}Te_{0,22}Si_{0,95}O_x$.

Préparation d'une solution de niobium :

Dans un bécher de 5 litres sont introduits :

- 640 g d'eau distillée,
- puis 51,2 g d'acide niobique, soit $n_{Nb}$ = 0,304 moles ;
- et enfin 103,2g d'acide oxalique dihydrate, soit $n_{oxalate}$ = 0,816 moles.

Le rapport molaire acide oxalique / Nb est dans ce cas de 2,69.
Le mélange est chauffé à 60 °C (un verre de montre est posé sur le bécher pour éviter l'évaporation) pendant 2 heures sous agitation. Une suspension blanche est obtenue. Le mélange est laissé refroidir jusqu'à 30°C sous agitation (environ 2 heures de refroidissement).
Parallèlement, une solution de Mo, V, Te est préparée de la façon suivante :

Dans un bécher de 5 litres sont introduits :

- 2120 g d'eau distillée,
- puis 488,0 g d'heptamolybdate d'ammonium, soit $n_{Mo}$ = 2,768 moles ;
- puis 106,4 g de métavanadate d'ammonium, soit $n_V$ = 0,912 moles ;
- et enfin 139,2 g d'acide tellurique, soit $n_{Te}$ = 0,608 moles.

Le mélange est chauffé à 60 °C (un verre de montre est posé sur le bécher pour éviter l'évaporation) pendant 1 heure 20 min et une solution limpide rouge est obtenue. Le mélange est laissé refroidir jusqu'à 30 °C tout en agitant (2 heures de refroidissement).
Introduction de la silice :

393,6 g de silice Ludox (40 % en poids de silice AS40) sont introduits, sous agitation, dans la solution de Mo, V, Te préparée comme ci-dessus. La solution reste limpide et rouge, mais un peu plus diluée. La solution de niobium est

introduite dans celle de (Mo, V, Te, Si) et un gel orange fluo est obtenu après quelques minutes d'agitation. Cette solution est alors séchée par atomisation (atomiseur de laboratoire - ATSELAB de la société Sodeva). L'atomisation se déroule sous atmosphère d'azote.

Les paramètres de marche sont globalement :

- débit d'azote de l'ordre de 45 Nm3/h ;
- débit de barbotine de l'ordre de 500 g/h ;
- température d'entrée des gaz comprise entre 155°C et 170°C ;
- température de sortie des gaz comprise entre 92°C et 100°C.

Le produit de granulométrie inférieure à 40 microns (355,2 g), récupéré dans le cyclone, est mis à l'étuve une nuit à 130°C, dans un plateau tefloné. 331 g de produit sec sont récupérés.

*Pré-calcination et calcination:*

Les 331 g de précurseur sont pré-calcinés pendant 4 heures à 300°C sous flux d'air (47,9 ml / min / g), ce qui donne un solide qui est calciné pendant 2 heures à 600 °C sous flux d'azote (12,8 ml / min / g). Le catalyseur A est ainsi obtenu.

Les calcinations sont effectuées sous flux d'air et d'azote dans des capacités en acier. Ces capacités sont directement installées dans les fours à moufles, et l'alimentation en air se fait par la cheminée. Un puits thermométrique interne permet un juste contrôle de la température. Le couvercle est utile dans le cas des calcinations sous azote pour éviter un retour d'air vers le catalyseur.

**Exemple B : Préparation d'un catalyseur B de formule : $Mo_1V_{0,30}Sb_{0,15}Nb_{0,10}Si_1O_x$ et de son précurseur symbolisé par la formule :**

$$Mo_1V_{0,30}Sb_{0,15}Nb_{0,10}(Oxalate)_{0,30}Si_1(H_2O_2)_{0,15}(NH_4)_{1,16}O_x$$

Synthèse du Précurseur : environ 100 g de précurseur sec sont ainsi préparés.

*Etape 1 : Dissolution-précipitation*

Solution A

12,3 g (0,1052 mol V) de métavanadate d'ammonium (MVA) (producteur GfE), 7,7 g (0,0528 mol Sb) de $Sb_2O_3$ (Producteur CAMPINE), 61,8 g d'heptamolybdate d'ammonium (HMA, 0,3501 mole de Mo) (Producteur Starck) sont mis en solution dans 130 ml d'eau déminéralisée, sous agitation, dans un réacteur SVL® en verre de 1 litre, chauffé dans un bain d'huile thermostaté à 128°C. Un léger ajout d'eau de 20 ml est nécessaire pour rincer l'entonnoir. Après l'ajout d'HMA, le réacteur est mis sous balayage d'azote, la réaction est maintenue sous agitation, à reflux, pendant 4 heures. Une suspension jaune est obtenue qui vire progressivement au bleu - noir.

Solution B

6 g (0,0530 mol) d'une solution aqueuse d'$H_2O_2$ à 30 % poids, dissous dans 50 g d'eau, sont alors ajoutés lentement (1 à 2 minutes environ). Afin d'obtenir une solution orange limpide, 8 gouttes d'eau oxygénée pure sont rajoutées.

Solution C

52,6 g de silice Ludox® AS40 ($n_{Si}$ = 0,350 mole) sont ajoutés en une fois. La solution se trouble légèrement. La solution formée est appelée solution C.

Solution D

Une solution D est préparée simultanément à la solution A. Dans un bêcher de 500 ml, on introduit 100 g d'eau distillée, 5,9 g d'acide niobique commercialisé par la société brésilienne CBMM soit $n_{Nb}$ = 0,035 mole, et 13,2 g d'acide oxalique Prolabo soit $n_{Oxalates}$ = 0,105 mole. Le mélange est chauffé à 70°C sous agitation pendant 2 heures, puis ramené à 30°C. La solution est ensuite centrifugée à 6200 tours/min pendant 12 minutes pour obtenir une solution limpide.

La solution D est ajoutée à la solution C, en une fois. On obtient un gel fluide orange puis jaune. L'agitation est maintenue pendant 30 minutes sous flux d'azote, sous reflux.

*Etape 2 : Séchage*

Le gel est alors séché en étuve ventilée, sur des plateaux recouverts de Téflon®, pendant la nuit, à 130°C. 111,4 g de précurseur sec sont récupérés. Le précurseur se présente sous forme de feuilles, noir dessus et pellicule mince verte en dessous. On obtient ainsi un précurseur symbolisé par la formule suivante qui reprend les principaux constituants : $Mo_1V_{0,30}Sb_{0,15}Nb_{0,10}(Oxalate)_{0,30}Si_1(H_2O_2)_{0,15}(NH_4)_{1,16}O_x$.

*Etape 3 : Traitement thermique*

30 g de précurseur otenu précédemment sont précalcinés à 317°C sous air statique.

Après calcination, à 594°C sous un débit d'azote de 49,8 ml/min/g, on obtient une masse de solide calciné de 24,4 g. Ce catalyseur est appelé CATALYSEUR B.

**Oxydation partielle du propane**

Dans les exemples qui suivent, les rendements, les sélectivités et la conversion du propane sont définies comme suit :

Rendement en acide acrylique (TTU) (%) = nombre de moles d'acide acrylique formées/nombre de moles de propane introduites x 100.

(Les rendements tiennent compte du nombre de mole de carbone dans chacun des produits et correspondent de fait au nombre de mole de propane ayant réagit équivalant).

Conversion du propane = TTG (propane) = somme du rendement en chacun des produits.

$$\text{Conversion du propane (\%)} = \frac{\text{Nombre de moles de propane ayant réagi}}{\text{Nombre de moles de propane introduites}} \times 100$$

$$\begin{array}{l}\text{Sélectivité (\%)}\\ \text{en acide acrylique}\end{array} = \frac{\text{Nombre de moles d'acide acrylique formées}}{\text{Nombre de moles de propane ayant réagi}} \times 100$$

Les sélectivités relatives aux autres composés sont calculées de manière similaire.

Les analyses sont validées lorsque les bilans carbone (somme des rendements en tous les produits détectés par analyse y compris le propane) sont compris entre 95 et 105 %, et lorsque le nombre de moles d'acide mesurées par titrimétrie à la soude correspond au nombre de moles d'acide dosées par chromatographie, à 10 % près.

Chargement des réacteurs

Tous les réacteurs de laboratoire ont été chargés selon un protocole analogue, dont un exemple est repris en détail ici. Nous avons dilué le catalyseur avec 10 ml de carbure de silicium 0,125 mm.

Exemple de chargement du réacteur [7] (dans le cas des Tests 3 et 4) : voir Figure 2

Appareillage (exemple du chargement du réacteur selon le Test 1)

Les tests sont effectués dans un réacteur en lit fixe.

Un réacteur vertical de forme cylindrique et en pyrex est chargé, du bas vers le haut et comprend :

- une première hauteur de 2 ml de carbure de silicium sous forme de particules de 0,125 mm de diamètre,
- une deuxième hauteur de 2 ml de carbure de silicium sous forme de particules de 0,062 mm de diamètre,
- une troisième hauteur de 1,00 g de catalyseur sous forme de particules de 0,02 à 1 mm dilué avec 10 ml de carbure de silicium sous forme de particules de 0,125 mm de diamètre,
- une quatrième hauteur de 2 ml de carbure de silicium sous forme de particules de 0,062 mm de diamètre,
- une cinquième hauteur de 2 ml de carbure de silicium sous forme de particules de 0,125 mm de diamètre, et
- une sixième hauteur de carbure de silicium sous forme de particules de 1,19 mm de diamètre, de manière à remplir la totalité du réacteur.

Tableau 1 : Volume et masse de SiC et Catalyseur par ordre de chargement dans le réacteur

| Chargement | Bilans N° | SiC 0,125 mm | SiC 0,062 mm | Catalyseur | SiC 0,125 mm / dilution | SiC 0,062 mm | SiC 0,125 mm |
|---|---|---|---|---|---|---|---|
| Test 1 | 1 à 4 | 2 ml | 2 ml | Catalyseur A 1,005 g (v = 0,9 ml) | 10 ml | 2 ml | 2 ml |
| Test 2 | 1 à 5 | 2 ml | 2 ml | Catalyseur A 0,516 g (v = 0,5 ml) | 10 ml | 2 ml | 2 ml |
| Test 3 | 1 à 4 | 4 ml | - | Catalyseur B 1,026 g (v = 1ml) | 10 ml | - | 4 ml |
| Test 4 | 1 à 5 | 4 ml | - | Catalyseur B 0,563 g (v = 0,5 ml) | 10 ml | - | 4 ml |

Description des tests

Pour étudier les performances des catalyseurs A et B, nous avons utilisé des tests co-alimentés. Les principales caractéristiques de ces tests et les informations recueillies sont les suivantes :

- Bilan co-alimenté, en conditions standard : test de base pour comparer rapidement des catalyseurs en mode co-alimenté ; conditions de test (débits des différents gaz) : Propane / Oxygène / Hélium-Krypton / Eau = 0,829 / 0,877 / 4,267 / 4,234 (en NL/h). La température de test est de 380 °C, 400 et 420 °C. Avec ce type de test, le temps de contact du gaz sur le catalyseur est d'environ 0,35 s suivant la densité du catalyseur.
- Bilan co-alimenté, avec un débit d'hélium doublé : conditions de test : Propane / Oxygène / Hélium-Krypton / Eau = 0,829 / 0,877 / 8,44 / 4,234 (en NL/h). Les températures de test sont de 380°C pour 0,5 g de catalyseur et 420°C pour 1 g de catalyseur. Avec un test de ce type, les temps de contact du gaz sur le catalyseur sont de environ 0,13 s pour 0,5 g de catalyseur et de environ 0,25 s pour 1 g.
- Bilan co-alimenté, avec un débit d'hélium nul et six fois plus de propane : conditions de test : Propane / Oxygène / Hélium-Krypton / Eau = 5,108 / 0,877 / 0 / 4,234 (en NL/h). Les températures de test sont de 420 et 440°C pour 0,5 g de catalyseur. Avec un test de ce type, les temps de contact du gaz sur le catalyseur sont de environ 0,18 s pour 0,5 g de catalyseur.

Tests des catalyseurs

1) Mode opératoire

Le réacteur contenant le catalyseur est placé dans un four isotherme vertical permettant d'imposer la température de réaction. Le haut du réacteur est connecté à l'alimentation commune en gaz et en vapeur d'eau.

Mise en chauffe sous flux de gaz inerte He/Kr :

- du réacteur à 250°C,
- du vaporiseur à 200°C,
- amorçage électrique de la pompe à eau.

Quand le vaporiseur et le réacteur sont à la bonne température, la pompe à eau est activée. Quand la température est atteinte et que l'eau est présente à la sortie du réacteur, le propane et l'oxygène sont ajoutés à leurs valeurs nominales. La température du réacteur est réglée à la température souhaitée, selon le catalyseur. On laisse la température du réacteur et la température du Point-chaud se stabiliser pendant 30 min au minimum.

Le bilan est effectué en branchant un flacon laveur, stocké dans de la glace afin de piéger les produits condensables, à la sortie du réacteur, et connecté au μ-GC pour analyser en ligne les effluents incondensables. Les effluents condensables récupérés sont analysés sur un chromatographe HP 6890, ainsi que par titrage à la soude du nombre de moles d'acide formées.

**Résultats des tests**

Calculs et Définitions complémentaires :

La VVH est la Vitesse Volumique Horaire, et s'exprime en $h^{-1}$. Elle représente le rapport entre le débit de gaz entrant et le volume de catalyseur. Ce dernier est mesuré dans une éprouvette. Le débit de gaz entrant est exprimé en normaux litres (litres de gaz mesurés à 0°C et 1 atm) par heure.

Le temps de contact est calculé en prenant l'inverse de la VVH. Pour des raisons pratiques, il est exprimé en secondes.

Il est possible d'obtenir une forte VVH en augmentant le débit de gaz dans le réacteur. Cependant, dans ce cas, la perte de charge générée sur le lit de catalyseur augmente. Néanmoins la productivité du réacteur s'en trouve aussi augmentée.

En général, on préfère diminuer la masse de catalyseur, ce qui ne perturbe pas l'hydrodynamique du réacteur et ne conduit pas à une augmentation de la perte de charge.

On calcule la productivité en un produit P comme étant le nombre de moles de ce produit par kg de catalyseur et par seconde. Dans le cas présent, nous avons calculé la productivité en équivalent C3, c'est à dire le nombre de micromoles de propane qui ont réagi pour donner le nombre de micromoles de produit P, par kg de catalyseur et par seconde. Dans le cas de produits à 3 carbones, les deux productivités sont identiques. Le résultat est obtenu directement en multipliant le rendement en produit P par le débit de propane entrant et en divisant le tout par la masse de catalyseur.

**Test 1** avec 1 g de catalyseur A

4 bilans ont été effectués dans les conditions suivantes

Bilans 1 à 3 :

Les débits d'alimentation en gaz sont de propane/oxygène/Hélium-Krypton/vapeur d'eau : 0,829 / 0,877 / 4,267 / 4,234 en Nl/h. La température du réacteur est de 380°C, 400 et 420°C.

Bilan 4 : pour ce bilan le débit d'Hélium-Krypton a été doublé - Propane / Oxygène / Hélium-Krypton / Eau = 0,829 / 0,877 / 8,44 / 4,234 (en NL/h)-, et la température du réacteur a été maintenue à 420°C.

Tableau 2 : Rendements et Sélectivités du Test 1

| **TEST 1** | Bilan 1 | Bilan 2 | Bilan 3 | Bilan 4 |
|---|---|---|---|---|
| Temp. réact. (°C) | 380 | 400 | 420 | 420 |
| Temp. Point Chaud (°C) | 384,8 | 405 | 425 | 425 |
| durée de stabilisation : | 00:53 | 01:22 | 00:45 | 00:35 |
| Temps de contact (s) | 0,32 | 0,32 | 0,32 | 0,23 |
| Conditions Gaz (Propane/$O_2$/He-Kr/$H_2O$) | 10/10/45/45 | 10/10/45/45 | 10/10/45/45 | 10/10/9045 |

(suite)

| TEST 1 | Bilan 1 | Bilan 2 | Bilan 3 | Bilan 4 |
|---|---|---|---|---|
| **Rendements** | TTUc (%) | | | |
| Acétaldéhyde | 0,00 | 0,00 | 0,01 | 0,00 |
| Propanaldéhyde | 0,00 | 0,00 | 0,00 | 0,00 |
| Acétone | 0,06 | 0,06 | 0,04 | 0,03 |
| Acroléine | 0,00 | 0,01 | 0,01 | 0,01 |
| Alcool Allylique | 0,00 | 0,00 | 0,00 | 0,00 |
| Acrylate d'Allyle | 0,00 | 0,00 | 0,00 | 0,00 |
| Acide Acétique | 0,12 | 0,21 | 0,31 | 0,18 |
| Acide Propionique | 0,03 | 0,03 | 0,02 | 0,01 |
| Acide Acrylique | 1,37 | 2,32 | 3,69 | 2,54 |
| CO | 0,08 | 0,22 | 0,52 | 0,29 |
| $CO_2$ | 0,15 | 0,23 | 0,45 | 0,35 |
| Propylène | 2,36 | 3,02 | 3,71 | 3,24 |
| Propane | 96,5 | 94,8 | 94,8 | 93,1 |
| Bilan Carbone (%) | 100,7 | 100,8 | 103,5 | 99,7 |
| TTG=Somme des TTU | 4,2 | 6,1 | 8,8 | 6,7 |
| **Sélectivités (%)** | Sélectivité (%) | | | |
| Acétaldéhyde | 0,08 | 0,07 | 0,06 | 0,06 |
| Propanaldéhyde | 0,00 | 0,00 | 0,00 | 0,00 |
| Acétone | 1,51 | 0,92 | 0,49 | 0,47 |
| Acroléine | 0,10 | 0,14 | 0,16 | 0,18 |
| Alcool Allylique | 0,05 | 0,04 | 0,03 | 0,03 |
| Acrylate d'Allyle | 0,00 | 0,00 | 0,00 | 0,00 |
| Acide Acétique | 2,99 | 3,43 | 3,49 | 2,76 |
| Acide Propionique | 0,62 | 0,43 | 0,24 | 0,20 |
| Acide Acrylique | 32,68 | 38,09 | 42,14 | 38,06 |
| CO | 1,85 | 3,56 | 5,90 | 4,38 |
| $CO_2$ | 3,63 | 3,72 | 5,13 | 5,24 |
| Propylène | 56,5 | 49,6 | 42,4 | 48,6 |

Tableau 3: Productivités du Test 1

| | Bilan 1 | Bilan 2 | Bilan 3 | Bilan 4 |
|---|---|---|---|---|
| **Productivités** | µmoleC3/kg.s | µmoleC3/kg.s | µmoleC3/kg.s | µmoleC3/kg.s |
| Acétaldéhyde | 0,4 | 0,4 | 0,5 | 0,4 |
| Propanaldéhyde | 0,0 | 0,0 | 0,0 | 0,0 |
| Acétone | 6,4 | 5,7 | 4,4 | 3,2 |
| Acroléine | 0,4 | 0,9 | 1,5 | 1,2 |
| Alcool Allylique | 0,2 | 0,2 | 0,3 | 0,2 |

(suite)

| | Bilan 1 | Bilan 2 | Bilan 3 | Bilan 4 |
|---|---|---|---|---|
| Acrylate d'Allyle | 0,0 | 0,0 | 0,0 | 0,0 |
| Acide Acétique | 12,8 | 21,3 | 31,3 | 18,8 |
| Acide Propionique | 2,7 | 2,6 | 2,2 | 1,3 |
| Acide Acrylique | 139,7 | 236,9 | 377,4 | 259,4 |
| CO | 7,9 | 22,2 | 52,8 | 29,8 |
| $CO_2$ | 15,5 | 23,1 | 45,9 | 35,7 |
| Propylène | 241,5 | 308,6 | 379,4 | 331,4 |

**Test 2** avec 0,5 g de catalyseur A

5 bilans ont été effectués dans les conditions suivantes

Bilans 1 et 3 :

Les débits d'alimentation en gaz sont de propane/oxygène/Hélium-Krypton/vapeur d'eau : 0,829 / 0,877 / 4,267 / 4,234 en Nl/h. La température du réacteur est de 380°C et 420°C.

Bilan 2 : pour ce bilan le débit d'Hélium-Krypton a été doublé - Propane / Oxygène / Hélium-Krypton / Eau = 0,829 / 0,877 / 8,44 / 4,234 (en NL/h)-, et la température du réacteur a été maintenue à 380°C.

Bilans 4 et 5 : pour ces bilans, le débit d'Hélium-Krypton a été ramené à 0, et le débit de propane a été fortement augmenté ce qui donne les conditions suivantes : propane/oxygène/Hélium-Krypton/vapeur d'eau : 5,108 / 0,877 / 0 / 4,234 (en NL/h). La température du réacteur est de 420 et 440°C. Etant donnés les forts débits de propane, les productivités en différents produits s'en trouvent fortement améliorées.

Tableau 4: Rendements et Sélectivités du Test 2

| **TEST 2** | Bilan 1 | Bilan 2 | Bilan 3 | Bilan 4 | Bilan 5 |
|---|---|---|---|---|---|
| Temp. réact. (°C) | 380 | 380 | 420 | 420 | 440 |
| Temp. Point Chaud (°C) | 384,1 | 383,8 | 424,8 | 429,3 | 454 |
| durée de stabilisation : | 00:53 | 00:36 | 01:00 | 01:00 | 00:24 |
| Temps de contact (s) | 0,18 | 0,13 | 0,18 | 0,18 | 0,18 |
| Cond. Gaz (Propane/$O_2$/He-Kr/$H_2O$) | 10/10/45/45 | 10/10/9045 | 10/10/45/45 | 60/10/0/45 | 60/10/0/45 |
| **Rendements** | TTUc (%) | | | | |
| Acétaldéhyde | 0,00 | 0,00 | 0,00 | 0,00 | 0,01 |
| Propanaldéhyde | 0,00 | 0,00 | 0,00 | 0,00 | 0,00 |
| Acétone | 0,03 | 0,02 | 0,03 | 0,03 | 0,03 |
| Acroléine | 0,00 | 0,00 | 0,01 | 0,01 | 0,02 |
| Alcool Allylique | 0,00 | 0,00 | 0,00 | 0,00 | 0,00 |
| Acrylate d'Allyle | 0,00 | 0,00 | 0,00 | 0,00 | 0,00 |
| Acide Acétique | 0,04 | 0,02 | 0,12 | 0,08 | 0,13 |
| Acide Propionique | 0,01 | 0,01 | 0,01 | 0,01 | 0,01 |
| Acide Acrylique | 0,53 | 0,32 | 1,48 | 0,94 | 1,58 |
| CO | 0,00 | 0,00 | 0,14 | 0,11 | 0,27 |
| $CO_2$ | 0,13 | 0,13 | 0,21 | 0,10 | 0,23 |
| Propylène | 1,90 | 1,61 | 3,27 | 3,54 | 4,61 |

(suite)

| TEST 2 | Bilan 1 | Bilan 2 | Bilan 3 | Bilan 4 | Bilan 5 |
|---|---|---|---|---|---|
| Propane | 98,0 | 98,9 | 96,2 | 98,1 | 98,8 |
| Bilan Carbone (%) | 100,6 | 101,0 | 101,4 | 102,9 | 105,7 |
| TTG=Somme des TTU | 2,6 | 2,1 | 5,3 | 4,8 | 6,9 |
| **Sélectivités (%)** | Sélectivité (%) | | | | |
| Acétaldéhyde | 0,10 | 0,08 | 0,09 | 0,06 | 0,08 |
| Propanaldéhyde | 0,00 | 0,00 | 0,00 | 0,02 | 0,02 |
| Acétone | 1,15 | 0,82 | 0,55 | 0,56 | 0,38 |
| Acroléine | 0,10 | 0,10 | 0,23 | 0,24 | 0,29 |
| Alcool Allylique | 0,05 | 0,00 | 0,03 | 0,02 | 0,01 |
| Acrylate d'Allyle | 0,00 | 0,00 | 0,00 | 0,00 | 0,00 |
| Acide Acétique | 1,43 | 0,94 | 2,20 | 1,64 | 1,94 |
| Acide Propionique | 0,42 | 0,32 | 0,28 | 0,22 | 0,12 |
| Acide Acrylique | 19,9 | 15,2 | 28,0 | 19,5 | 22,9 |
| CO | 0,00 | 0,00 | 2,65 | 2,37 | 3,95 |
| $CO_2$ | 4,77 | 6,01 | 3,89 | 2,01 | 3,36 |
| Propylène | 72,1 | 76,6 | 62,1 | 73,4 | 66,9 |

Tableau 5 : Productivités du Test 2

| | Bilan 1 | Bilan 2 | Bilan 3 | Bilan 4 | Bilan 5 |
|---|---|---|---|---|---|
| **Productivités** | µmoleC3/kg.s | | | | |
| Acétaldéhyde | 0,5 | 0,3 | 1,0 | 3,6 | 6,5 |
| Propanaldéhyde | 0,0 | 0,0 | 0,0 | 1,1 | 1,3 |
| Acétone | 6,1 | 3,4 | 5,8 | 33,1 | 31,8 |
| Acroléine | 0,5 | 0,4 | 2,4 | 14,1 | 24,6 |
| Alcool Allylique | 0,2 | 0,0 | 0,3 | 1,0 | 1,2 |
| Acrylate d'Allyle | 0,0 | 0,0 | 0,0 | 0,0 | 0,0 |
| Acide Acétique | 7,5 | 3,9 | 23,1 | 97,0 | 164,3 |
| Acide Propionique | 2,2 | 1,3 | 2,9 | 13,2 | 10,5 |
| Acide Acrylique | 104,6 | 63,7 | 293,9 | 1155,6 | 1936,2 |
| CO | 0,0 | 0,0 | 27,8 | 140,2 | 334,1 |
| CO2 | 25,0 | 25,2 | 40,9 | 119,2 | 284,2 |
| Propylène | 378,3 | 321,5 | 652,0 | 4343,5 | 5656,3 |

Sur l'ensemble des tests Test 1, bilans 1 à 4 et Test 2, bilans 1 à 3, on observe que lorsque le temps de contact diminue, la sélectivité en propylène (produit majoritaire) augmente, et que la somme des sélectivités en acide acrylique + propylène augmente. Le catalyseur A, permet donc d'opérer avec une haute sélectivité en propylène et une forte sélectivité globale en acide acrylique + propylène, à basse conversion.

Les résultats des bilans 4 et 5 du Test 2, montrent que lorsque la pression partielle en propane est de l'ordre de 50 %, il est possible d'avoir de fortes productivités en propylène et en acide acrylique, tout en travaillant à basse conversion. Dans ce cas également les sélectivités en produits utiles restent particulièrement élevées.

<u>Test 3</u> avec 1 g de catalyseur B - Effectué comme le Test 1 mais avec un catalyseur à l'antimoine et non au tellure.

4 bilans ont été effectués dans les conditions suivantes :

<u>Bilans 1 à 3 :</u>

Les débits d'alimentation en gaz sont de propane/oxygène/Hélium-Krypton/vapeur d'eau : 0,829 / 0,877 / 4,267 / 4,234 (en NL/h). La température du réacteur est de 380, 400 et 420°C.
<u>Bilan 4:</u> pour ce bilan le débit d'Hélium-Krypton a été doublé - Propane / Oxygène / Hélium-Krypton / Eau = 0,829 / 0,877 / 8,44 / 4,234 (en NL/h), et la température du réacteur a été maintenue à 420°C.

Tableau 6: Rendements et Sélectivités du Test 3

| **TEST 3** | Bilan 1 | Bilan 2 | Bilan 3 | Bilan 4 |
|---|---|---|---|---|
| Temp. réact. (°C) | 380 | 400 | 420 | 420 |
| Temp. Point Chaud (°C) | 386 | 406,6 | 427 | 426 |
| durée de stabilisation : | 00:55 | 01:00 | 00:45 | 00:35 |
| Temps de contact (s) | 0,35 | 0,35 | 0,35 | 0,25 |
| Cond. Gaz (Propane/$O_2$/He-Kr/$H_2O$) | 10/10/45/45 | 10/10/45/45 | 10/10/45/45 | 10/10/9045 |
| **Rendements** | TTUc (%) | | | |
| Acétaldéhyde | 0,00 | 0,00 | 0,01 | 0,00 |
| Propanaldéhyde | 0,00 | 0,00 | 0,00 | 0,00 |
| Acétone | 0,10 | 0,09 | 0,07 | 0,05 |
| Acroléine | 0,01 | 0,01 | 0,02 | 0,01 |
| Alcool Allylique | 0,00 | 0,00 | 0,00 | 0,00 |
| Acrylate d'Allyle | 0,00 | 0,00 | 0,00 | 0,00 |
| Acide Acétique | 0,18 | 0,29 | 0,39 | 0,22 |
| Acide Propionique | 0,03 | 0,03 | 0,02 | 0,01 |
| Acide Acrylique | 1,67 | 2,56 | 3,60 | 2,40 |
| CO | 0,18 | 0,33 | 0,55 | 0,29 |
| $CO_2$ | 0,19 | 0,28 | 0,43 | 0,27 |
| Propylène | 2,99 | 3,58 | 4,17 | 3,59 |
| Propane | 96,7 | 93,9 | 91,5 | 92,9 |
| Bilan Carbone (%) | 102,1 | 101,1 | 100,8 | 99,7 |
| TTG=Somme des TTU | 5,4 | 7,2 | 9,3 | 6,9 |
| **Sélectivités (%)** | Sélectivités (%) | | | |
| Acétaldéhyde | 0,06 | 0,06 | 0,07 | 0,06 |
| Propanaldéhyde | 0,00 | 0,00 | 0,00 | 0,00 |
| Acétone | 1,83 | 1,24 | 0,78 | 0,73 |
| Acroléine | 0,14 | 0,14 | 0,17 | 0,19 |
| Alcool Allylique | 0,00 | 0,00 | 0,00 | 0,00 |
| Acrylate d'Allyle | 0,00 | 0,00 | 0,00 | 0,00 |
| Acide Acétique | 3,43 | 4,02 | 4,18 | 3,16 |
| Acide Propionique | 0,65 | 0,43 | 0,26 | 0,21 |

(suite)

| TEST 3 | Bilan 1 | Bilan 2 | Bilan 3 | Bilan 4 |
|---|---|---|---|---|
| Acide Acrylique | 31,2 | 35,7 | 38,9 | 35,1 |
| CO | 3,33 | 4,62 | 5,93 | 4,17 |
| $CO_2$ | 3,51 | 3,95 | 4,65 | 3,97 |
| Propylène | 55,8 | 49,9 | 45,0 | 52,4 |

Tableau 7: Productivités du Test 3

| | Bilan 1 | Bilan 2 | Bilan 3 | Bilan 4 |
|---|---|---|---|---|
| **Productivités** | µmoleC3/kg.s | | | |
| Acétaldéhyde | 0,34 | 0,41 | 0,68 | 0,42 |
| Propanaldéhyde | 0,00 | 0,00 | 0,00 | 0,00 |
| Acétone | 9,79 | 8,91 | 7,27 | 5,00 |
| Acroléine | 0,73 | 1,00 | 1,56 | 1,33 |
| Alcool Allylique | 0,00 | 0,00 | 0,00 | 0,00 |
| Acrylate d'Allyle | 0,00 | 0,00 | 0,00 | 0,00 |
| Acide Acétique | 18,41 | 28,90 | 38,74 | 21,72 |
| Acide Propionique | 3,48 | 3,07 | 2,40 | 1,46 |
| Acide Acrylique | 167,58 | 256,34 | 361,07 | 240,97 |
| CO | 17,85 | 33,20 | 55,02 | 28,64 |
| $CO_2$ | 18,83 | 28,41 | 43,15 | 27,27 |
| Propylène | 299,31 | 358,74 | 417,46 | 360,19 |

Test 4 avec 0,5 g de catalyseur A - Effectué comme le Test 2 mais avec un catalyseur à l'antimoine

5 bilans ont été effectués dans les conditions suivantes

Bilans 1 et 3 :

Les débits d'alimentation en gaz sont de propane/oxygène/Hélium-Krypton/vapeur d'eau : 0,829 / 0,877 / 4,267 / 4,234 (en NL/h). La température du réacteur est de 380 et 420°C.

Bilan 2 : pour ce bilan le débit d'Hélium-Krypton a été doublé - Propane / Oxygène / Hélium-Krypton / Eau = 0,829 / 0,877 / 8,44 / 4,234 (en NL/h), et la température du réacteur a été maintenue à 380°C.

Bilans 4 et 5 : pour ces bilans, le débit d'Hélium-Krypton a été ramené à 0, et le débit de propane a été fortement augmenté ce qui donne les conditions suivantes : propane/oxygène/Hélium-Krypton/vapeur d'eau : 5,108 / 0,877 / 0 / 4,234 (en NL/h). La température du réacteur est de 420 et 440°C. Etant donnés les forts débits de propane, les productivités en différents produits s'en trouvent fortement améliorées.

Tableau 8: Rendements et Sélectivités du Test 4

| TEST 4 | Bilan 1 | Bilan 2 | Bilan 3 | Bilan 4 | Bilan 5 |
|---|---|---|---|---|---|
| Temp. réact. (°C) | 380 | 380 | 420 | 420 | 440 |
| Temp. Point Chaud (°C) | 386 | 385,8 | 426,6 | 428,3 | 449,7 |
| durée de stabilisation : | 00 :53 | 00:36 | 01:00 | 00:32 | 00:24 |
| Temps de contact (s) | 0,18 | 0,13 | 0,18 | 0,18 | 0,18 |

(suite)

| TEST 4 | Bilan 1 | Bilan 2 | Bilan 3 | Bilan 4 | Bilan 5 |
|---|---|---|---|---|---|
| Cond. Gaz (Propane/$O_2$/He-Kr/$H_2O$) | 10/10/45/45 | 10/10/9045 | 10/10/45/45 | 60/10/0/45 | 60/10/0/45 |
| **Rendements** | TTUc (%) | | | | |
| Acétaldéhyde | 0,01 | 0,00 | 0,01 | 0,00 | 0,01 |
| Propanaldéhyde | 0,00 | 0,00 | 0,00 | 0,00 | 0,00 |
| Acétone | 0,03 | 0,02 | 0,05 | 0,03 | 0,03 |
| Acroléine | 0,00 | 0,00 | 0,01 | 0,01 | 0,02 |
| Alcool Allylique | 0,00 | 0,00 | 0,00 | 0,00 | 0,00 |
| Acrylate d'Allyle | 0,00 | 0,00 | 0,00 | 0,00 | 0,00 |
| Acide Acétique | 0,04 | 0,03 | 0,17 | 0,07 | 0,10 |
| Acide Propionique | 0,01 | 0,00 | 0,01 | 0,01 | 0,01 |
| Acide Acrylique | 0,57 | 0,43 | 1,84 | 0,71 | 0,95 |
| CO | 0,00 | 0,00 | 0,29 | 0,10 | 0,17 |
| $CO_2$ | 0,13 | 0,15 | 0,29 | 0,08 | 0,14 |
| Propylène | 2,05 | 1,88 | 3,68 | 3,27 | 3,97 |
| Propane | 98,7 | 97,4 | 94,6 | 98,6 | 98,7 |
| Bilan Carbone (%) | 101,6 | 99,9 | 101,0 | 102,9 | 104,1 |
| TTG=Somme des TTU | 2,9 | 2,5 | 6,4 | 4,3 | 5,4 |
| **Sélectivités (%)** | Sélectivités (%) | | | | |
| Acétaldéhyde | 0,21 | 0,12 | 0,16 | 0,09 | 0,11 |
| Propanaldéhyde | 0,00 | 0,00 | 0,00 | 0,02 | 0,03 |
| Acétone | 1,04 | 0,70 | 0,71 | 0,73 | 0,50 |
| Acroléine | 0,16 | 0,14 | 0,23 | 0,25 | 0,29 |
| Alcool Allylique | 0,04 | 0,00 | 0,00 | 0,01 | 0,01 |
| Acrylate d'Allyle | 0,00 | 0,00 | 0,00 | 0,00 | 0,00 |
| Acide Acétique | 1,45 | 1,08 | 2,65 | 1,69 | 1,90 |
| Acide Propionique | 0,38 | 0,20 | 0,23 | 0,27 | 0,16 |
| Acide Acrylique | 20,1 | 17,2 | 29,0 | 16,6 | 17,6 |
| CO | 0,00 | 0,00 | 4,57 | 2,29 | 3,19 |
| $CO_2$ | 4,63 | 5,86 | 4,50 | 1,96 | 2,65 |
| Propylène | 72,0 | 74,7 | 58,0 | 76,1 | 73,5 |

Tableau 9: Productivités du Test 4

| | Bilan 1 | Bilan 2 | Bilan 3 | Bilan 4 | Bilan 5 |
|---|---|---|---|---|---|
| **Productivités** | µmoleC3/kg.s | | | | |
| Acétaldéhyde | 1,1 | 0,6 | 1,8 | 4,2 | 6,6 |
| Propanaldéhyde | 0,0 | 0,0 | 0,0 | 1,2 | 2,1 |
| Acétone | 5,4 | 3,2 | 8,2 | 35,3 | 30,3 |
| Acroléine | 0,8 | 0,7 | 2,7 | 11,9 | 17,4 |

(suite)

| | Bilan 1 | Bilan 2 | Bilan 3 | Bilan 4 | Bilan 5 |
|---|---|---|---|---|---|
| Alcool Allylique | 0,2 | 0,0 | 0,0 | 0,5 | 0,5 |
| Acrylate d'Allyle | 0,0 | 0,0 | 0,0 | 0,0 | 0,0 |
| Acide Acétique | 7,5 | 4,9 | 30,7 | 81,8 | 115,4 |
| Acide Propionique | 2,0 | 0,9 | 2,7 | 12,8 | 9,7 |
| Acide Acrylique | 104,9 | 78,9 | 336,4 | 801,6 | 1073,3 |
| CO | 0,0 | 0,0 | 53,0 | 110,8 | 194,2 |
| $CO_2$ | 24,1 | 26,9 | 52,3 | 94,9 | 160,9 |
| Propylène | 374,9 | 342,6 | 672,8 | 3675,9 | 4472,1 |

Sur l'ensemble des tests Test 3, bilans 1 à 4 et Test 4, bilans 1 à 3, on observe que lorsque le temps de contact diminue, la sélectivité en propylène (produit majoritaire) augmente, et que la somme des sélectivités en acide acrylique + propylène augmente. Le catalyseur B, permet donc d'opérer avec une haute sélectivité en propylène et une forte sélectivité globale en acide acrylique + propylène, à basse conversion.

Les résultats des bilans 4 et 5 du Test 4, montrent que lorsque la pression partielle en propane est de l'ordre de 50 %, il est possible d'avoir de fortes productivités en propylène et en acide acrylique, tout en travaillant à basse conversion. D'autre part, dans ce cas aussi les sélectivités en produits utiles restent particulièrement élevées.

On constate également que les sélectivités en acide propionique et acétone diminuent avec l'augmentation de température.

## Revendications

1. Un procédé de préparation de l'acide acrylique par oxydation du propylène puis de l'acroléine, **caractérisé en ce que** l'on met en oeuvre le recyclage des gaz n'ayant pas réagi, en effectuant une oxydation partielle du propane, en parallèle, à l'issue de l'étape de récupération de l'acide acrylique, puis en retournant vers le réacteur de conversion du propylène, un gaz riche en propane et en propylène ayant subi un second passage dans la colonne de récupération de l'acide acrylique.

2. Un procédé de préparation selon la revendication 1, **caractérisé en ce que** le propane est soumis à une conversion partielle en propylène, dans un réacteur placé en parallèle de la colonne d'adsorption destinée à la récupération de l'acide acrylique, puis le flux gazeux est réintroduit dans la colonne d'absorption utilisée pour la récupération de l'acide acrylique.

3. Un procédé de préparation selon l'une des revendications 1 ou 2, **caractérisé en ce que** le propane est soumis à une conversion partielle en propylène, en présence d'un catalyseur constitué de mélange d'oxydes.

4. Un procédé de préparation selon l'une des revendications 1 à 3, **caractérisé en ce que** le procédé d'oxydation partielle du propane en propylène est mis en oeuvre à une température de 300 à 450°C et de préférence supérieure à 380°C.

5. Un procédé de préparation selon l'une des revendications 1 à 4, **caractérisé en ce que** le procédé d'oxydation partielle du propane en propylène est mis en oeuvre à des vitesses volumiques horaires élevées VVH supérieures à 10000 $h^{-1}$ et de préférence comprises entre des valeurs supérieures à 10 000 $h^{-1}$, et 20 000 $h^{-1}$.

6. Un procédé de préparation selon l'une des revendications 1 à 5, **caractérisé en ce que** le procédé d'oxydation partielle du propane en propylène est mis en oeuvre en présence d'un catalyseur, ledit catalyseur étant un mélange d'oxydes contenant de éléments choisis parmi le molybdène, le vanadium, le tellure, le niobium, le tantale, le silicium et/ou l'antimoine.

7. Un procédé de préparation selon la revendication 6, **caractérisé en ce que** le catalyseur répond à la structure :

$$Mo_1V_a(Te\ ou\ Sb)_b(Nb\ ou\ Ta)_cSi_dO_x \qquad (I)$$

dans laquelle :

- a est compris entre 0,006 et 1, bornes incluses ;
- b est compris entre 0,006 et 1, bornes incluses ;
- c est compris entre 0,006 et 1, bornes incluses ;
- d est compris entre 0 et 3,5, bornes incluses ; et
- x est la quantité d'oxygène lié aux autres éléments et dépend de leurs états d'oxydation.

**8.** Un procédé de préparation selon la revendication 7, **caractérisé en ce que** le catalyseur répond à la structure (Ia):

$$Mo_1V_aSb_bNb_cSi_dO_x \qquad (Ia)$$

dans laquelle a, b, c, d et x sont définis comme dans la revendication 7.

**9.** Un procédé de préparation selon l'une des revendications 7 ou 8, **caractérisé en ce que** le catalyseur répond respectivement à la structure (I) ou (Ia) dans laquelle :

- a est compris entre 0,01 et 0,6, bornes incluses ;
- b est compris entre 0,01 et 0,5, bornes incluses ;
- c est compris entre 0,006 et 0,3, bornes incluses ;
- d est compris entre 0 et 2, bornes incluses ; et

x est la quantité d'oxygène lié aux autres éléments et dépend de leurs états d'oxydation.

**10.** Un dispositif destiné à la préparation de l'acide acrylique **caractérisé en ce qu'**il comprend :

a) un premier réacteur d'oxydation du propylène en acroléine [3], alimentant en continu
b) un second réacteur destiné à l'oxydation de l'acroléine en acide acrylique [4], relié à
c) une colonne d'absorption [5] destinée à la récupération de l'acide acrylique, les gaz n'ayant pas réagi étant dirigés vers
d) un réacteur [7] d'oxydation partielle du propane en propylène, placé en parallèle à la sortie de la colonne d'absorption [5], lesdits gaz subissant à la sortie un nouveau passage dans la colonne d'absorption ou dans une colonne similaire, puis
e) un recyclage dans le premier réacteur d'oxydation du propylène [3].

**11.** Utilisation du procédé selon l'une des revendications 1 à 9, pour la préparation de l'acide acrylique.

## Claims

**1.** A process for the preparation of acrylic acid by oxidation of propylene then acrolein, **characterized in that** recycling of the unreacted gases is implemented by carrying out a partial oxidation of propane, in parallel at the outlet of the acrylic acid recovery stage, then by returning to the propylene conversion reactor a gas rich in propane and propylene having undergone a second pass through the acrylic acid recovery column.

**2.** A preparation process according to claim 1, **characterized in that** the propane undergoes partial conversion to propylene, in a reactor arranged in parallel to the adsorption column intended for the recovery of acrylic acid, then the gas flow is reintroduced into the absorption column used for the recovery of acrylic acid.

**3.** A preparation process according to one of claims 1 or 2, **characterized in that** the propane undergoes a partial conversion to propylene, in the presence of a catalyst constituted by a mixture of oxides.

**4.** A preparation process according to one of claims 1 to 3, **characterized in that** the process of partial oxidation of propane to propylene is implemented at a temperature of 300 to 450°C and preferably greater than 380°C.

**5.** A preparation process according to one of claims 1 to 4, **characterized in that** the process of partial oxidation of

propane to propylene is implemented at high volume velocities per hour VVH greater than 10000 $h^1$ and preferably comprised between values exceeding 10 000 $h^4$, and 20 000 $h^1$.

6. A preparation process according to one of claims 1 to 5, **characterized in that** the process of partial oxidation of the propane to propylene is implemented in the presence of a catalyst, said catalyst being a mixture of oxides containing elements chosen from molybdenum, vanadium, tellurium, niobium, tantalum, silicon and/or antimony.

7. A preparation process according to claim 6, **characterized in that** the catalyst corresponds to the structure:

$$Mo_1V_a(Te\ or\ Sb)_b(Nb\ or\ Ta)_cSi_dOx \qquad (I)$$

in which:

- a is comprised between 0.006 and 1, inclusive;
- b is comprised between 0.006 and 1, inclusive;
- c is comprised between 0.006 and 1, inclusive;
- d is comprised between 0 and 3.5, inclusive; and
- x is the quantity of oxygen bound to the other elements and depends on their oxidation states.

8. A preparation process according to claim 7, **characterized in that** the catalyst corresponds to the structure:

$$Mo_1V_aSb_6NbcSi_dOx \qquad (Ia)$$

in which a, b, c, d and x are defined as in claim 7.

9. A preparation process according to one of claims 7 or 8, **characterized in that** the catalyst corresponds to structure (I) or (Ia) respectively in which:

- a is comprised between 0.01 and 0.6, inclusive;
- b is comprised between 0.01 and 0.5, inclusive;
- c is comprised between 0.006 and 0.3, inclusive;
- d is comprised between 0 and 2, inclusive; and

x is the quantity of oxygen bound to the other elements and depends on their oxidation states.

10. A device intended for the preparation of acrylic acid, **characterized in that** it comprises:

a) a first, continuous feed, reactor for the oxidation of propylene to acrolein [3],
b) a second reactor intended for the oxidation of acrolein to acrylic acid [4], connected to
c) an absorption column [5] intended for the recovery of acrylic acid, the unreacted gases being routed to
d) a reactor for the partial oxidation of propane to propylene [7], arranged in parallel with the outlet of the absorption column [5], said gases undergoing at the outlet a further pass through the absorption column or a similar column, then
e) recycling into the first propylene oxidation reactor [3].

11. Use of the process according to one of claims 1 to 9, for the preparation of acrylic acid.

**Patentansprüche**

1. Verfahren zur Herstellung von Acrylsäure durch Oxidation zunächst von Propylen und dann von Acrolein, **dadurch gekennzeichnet, dass** die Gase, welche nicht umgesetzt wurden, zurückgeführt werden, wobei parallel dazu, nach dem Schritt der Gewinnung der Acrylsäure, eine teilweise Oxidation des Propans durchgeführt wird und anschließend ein Gas, das reich an Propan ist sowie an Propylen, welches ein zweites Mal durch die Kolonne zur Gewinnung der Acrylsäure geströmt ist, in den Reaktor zur Umwandlung des Propylen zurückgeführt wird.

2. Herstellungsverfahren nach Anspruch 1, **dadurch gekennzeichnet, dass** das Propan in einem Reaktor, der parallel zur Adsorptionskolonne, welche zur Gewinnung der Acrylsäure bestimmt ist, angeordnet ist, einer teilweisen Umwandlung in Propylen unterzogen wird, woraufhin der Gasstrom erneut in die Absorptionskolonne, die zur Gewinnung

der Acrylsäure verwendet wird, eingeleitet wird.

3. Herstellungsverfahren nach einem der Ansprüche 1 oder 2, **dadurch gekennzeichnet, dass** das Propan in Gegenwart eines Katalysators, der aus Mischoxid besteht, einer teilweisen Umwandlung in Propylen unterzogen wird.

4. Herstellungsverfahren nach einem der Ansprüche 1 bis 3, **dadurch gekennzeichnet, dass** das Verfahren zur teilweisen Oxidation des Propans zu Propylen bei einer Temperatur von 300 bis 450 °C und vorzugsweise von mehr als 380 °C durchgeführt wird.

5. Herstellungsverfahren nach einem der Ansprüche 1 bis 4, **dadurch gekennzeichnet, dass** das Verfahren zur teilweisen Oxidation des Propans zu Propylen mit hohen stündlichen Volumendurchsatzraten von mehr als 10.000 $h^{-1}$ durchgeführt wird, wobei diese vorzugsweise zwischen Werten von über 10.000 $h^{-1}$ und 20.000 $h^{-1}$ liegen.

6. Herstellungsverfahren nach einem der Ansprüche 1 bis 5, **dadurch gekennzeichnet, dass** das Verfahren zur teilweisen Oxidation des Propans zu Propylen in Gegenwart eines Katalysators durchgeführt wird, wobei der Katalysator ein Mischoxid ist, das Elemente enthält, die aus Molybdän, Vanadium, Tellur, Niob, Tantal, Silicium und/oder Antimon gewählt sind.

7. Herstellungsverfahren nach Anspruch 6, **dadurch gekennzeichnet, dass** der Katalysator die folgende Struktur aufweist:

$$Mo_1V_a(\text{Te oder Sb})_b(\text{Nb oder Ta})_cSi_dO_x \qquad (I)$$

wobei:

- a: 0,006 bis 1 beträgt, einschließlich der Grenzwerte;
- b: 0,006 bis 1 beträgt, einschließlich der Grenzwerte;
- c: 0,006 bis 1 beträgt, einschließlich der Grenzwerte;
- d: 0 bis 3,5 beträgt, einschließlich der Grenzwerte;
und
- x: die Menge an Sauerstoff ist, die an die weitere Elemente gebunden ist, und von derenOxidationszahl abhängt

8. Herstellungsverfahren nach Anspruch 7, **dadurch gekennzeichnet, dass** der Katalysator die folgende Struktur (Ia) aufweist:

$$Mo_1V_aSb_bNb_cSi_dO_x \qquad (Ia)$$

wobei a, b, c, d et x den jeweiligen Definitionen in Anspruch 7 entsprechen.

9. Herstellungsverfahren nach einem der Ansprüche 7 oder 8, **dadurch gekennzeichnet, dass** der Katalysator die Struktur (I) beziehungsweise (Ia) aufweist, wobei:

- a: 0,01 bis 0,6 beträgt, einschließlich der Grenzwerte;
- b: 0,01 bis 0,5 beträgt, einschließlich der Grenzwerte;
- c: 0,006 bis 0,3 beträgt, einschließlich der Grenzwerte;
- d: 0 bis 2 beträgt, einschließlich der Grenzwerte; und
- x: die Menge an Sauerstoff ist, die an die weiteren Elemente gebunden ist, und von deren Oxidationszahl abhängt.

10. Vorrichtung, die zur Herstellung von Acrylsäure bestimmt ist, **dadurch gekennzeichnet, dass** sie Folgendes umfasst:

a) einen ersten Reaktor [3] zur Oxidation des Propylens zu Acrolein, wobei dieser kontinuierlich Folgendes speist
b) einen zweiten Reaktor[4], der zur Oxidation des Acroleins zu Acrylsäure bestimmt ist und mit Folgendem verbunden ist
c) eine Absorptionskolonne [5] die zur Gewinnung der Acrylsäure bestimmt ist, wobei die Gase, welche nicht umgesetzt wurden, Folgendem zugeführt werden
d) einem Reaktor [7] zur teilweisen Oxidation des Propan zu Propylen, welcher an der Austrittsöffnung der

Absorptionskolonne [5] parallel angeordnet ist, wobei die Gase an der Austrittsöffnung erneut in die Absorptionskolonne oder in eine ähnliche Kolonne eingeleitet werden, woraufhin
e) sie in den ersten Reaktor [3] zur Oxidation des Propylens zurückgeführt werden.

**11.** Verwendung des Verfahrens nach einem der Ansprüche 1 bis 9 zur Herstellung von Acrylsäure.

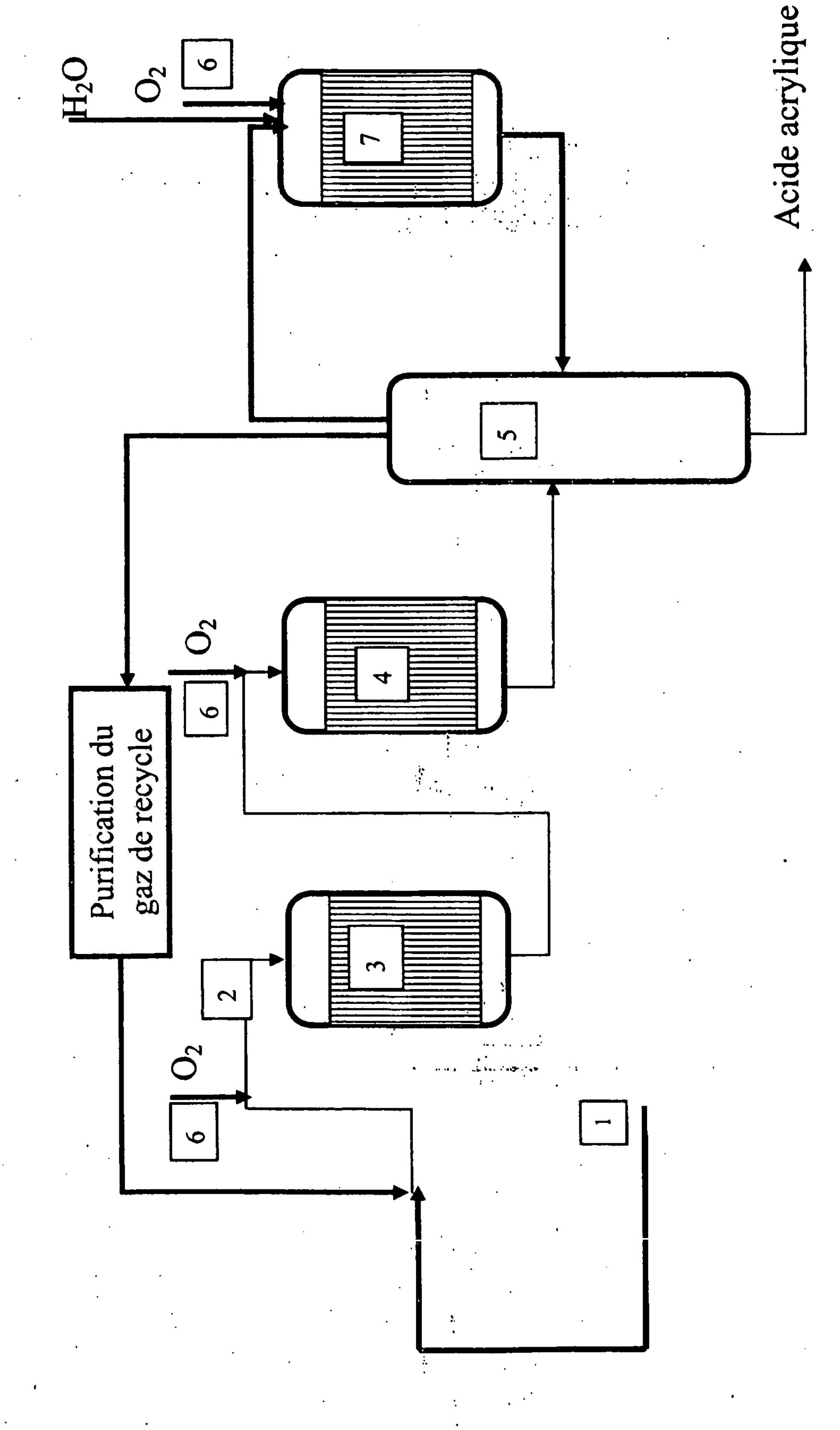

$H_2O$
$O_2$
6
7
Acide acrylique
5
$O_2$
6
4
Purification du gaz de recycle
3
2
$O_2$
6
1


Figure 1

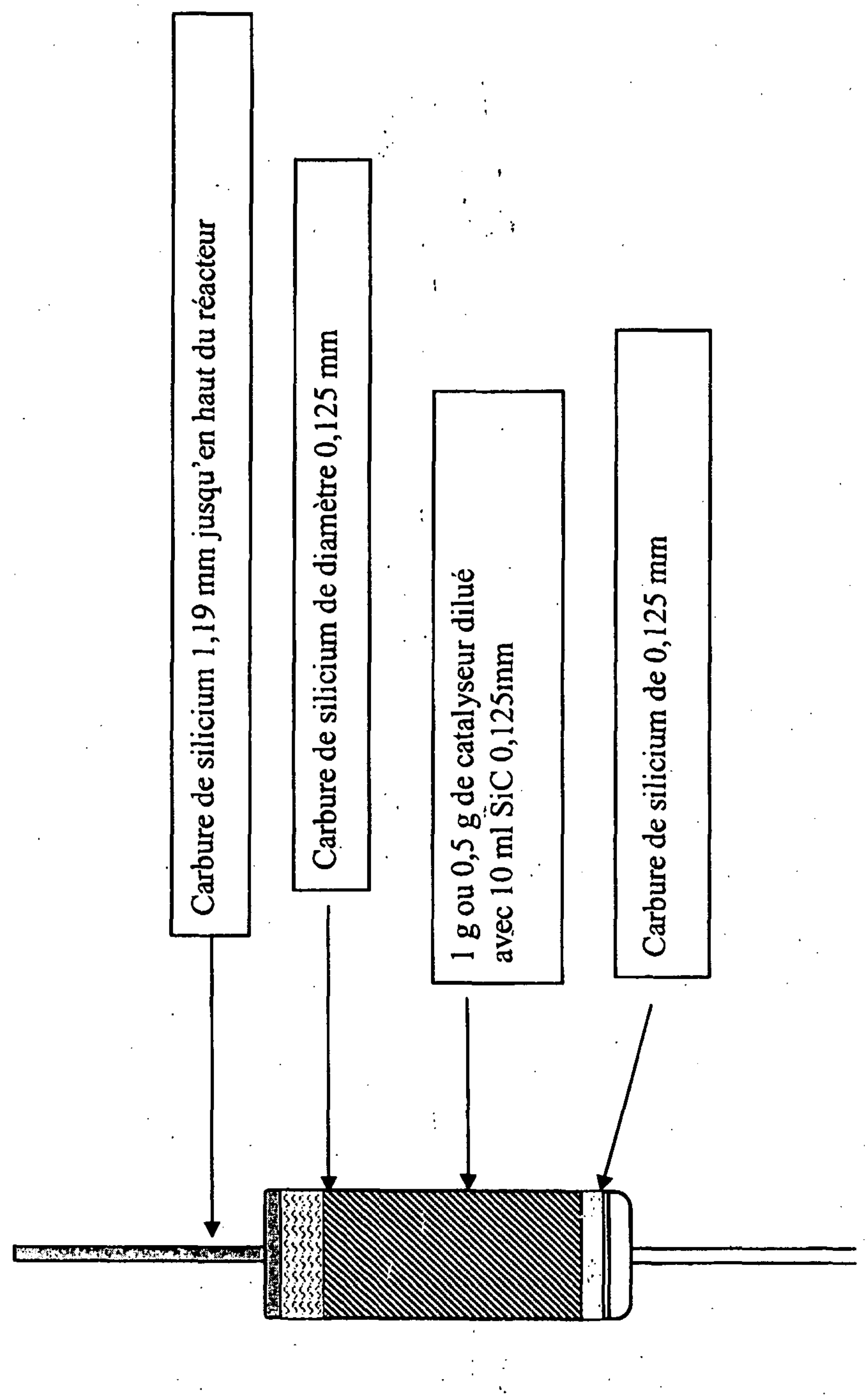
Carbure de silicium 1,19 mm jusqu'en haut du réacteur
Carbure de silicium de diamètre 0,125 mm
1 g ou 0,5 g de catalyseur dilué avec 10 ml SiC 0,125mm
Carbure de silicium de 0,125 mm

**Figure 2**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*


### Documents brevets cités dans la description

- EP 293224 A
- US 6492548 B

### Littérature non-brevet citée dans la description

- **L. Luo ; J. A. Labinger ; M. E. Davis.** *J. of Catalysis,* 2001, vol. 200, 222-231